# EUROPEAN PATENT APPLICATION

(11) **EP 1 839 673 A2**
(43) Date of publication of application: **03.10.2007**
(21) Application number: 07008760.6
(22) Date of filing: 14.05.1999
(51) Int. Cl.: A61K 39/385, C07K 7/06, C07K 7/08

(54) **Prevention and treatment of hypergastrinemia**

(30) Priority: 15.05.1998 US 85714 P
(62) Divisional of application: 99924258.9
(71) Applicant: Receptor Biologix, Inc., Palo Alto, California 94303 (US)
(72) Inventor: Gevas, Philip C., Key Biscayne, FL 33149 (US); Grimes, Stephen, Davis, CA 95616 (US); Karr, Stephen, Davis, CA 95616 (US); Michaeli, Dov, Larkspur, CA 94939 (US); Watson, Susan, Edwalton, Nottingham, NG2 6RB (GB)
(74) Representative: Manaton, Ross Timothy

(57) **Abstract**

Serum-associated hypergastrinemia is treated by administration of gastrin active or passive immunization. An anti-gastrin immunogenic composition comprising a gastrin G17 or G34 peptide fragment which is amino acid spacer-linked to an immunogenic carrier, is administered so as to effectively neutralize the circulating gastrin hormone, and moreover, inhibit autocrine activity by progastrin such as Gly-extended G17, and amidated G 17, in patients with pernicious anemia. Moreover, the method includes administration of a therapeutically effective amount of anti-G17 or anti-G34 antibodies which may be in humanized form. Finally, the method provides ameliorating treatment of hypergastrinemic effects of proton pump inhibitors or H2 histamine receptor blocking agents or antagonists, in addition to treatment of hypergastrinemia caused by diseases such as pernicious anemia.

## Description

### FIELD OF THE INVENTION

The invention relates to the prevention and/or treatment of hypergastrinemia by immunological control of gastrin levels.

### BACKGROUND OF THE INVENTION

In humans, treatment with proton pump inhibitors, infection with *Helicobacter pylori* and pernicious anemia account for the majority of cases of hypergastrinemia. Marked hypergastrinemia is seen in the relatively infrequent Zollinger-Ellison Syndrome (ZES). One of the direct effects of hypergastrinemia is, of course, high secretion rates of gastric acid in the stomach.

Around 90% of patients with pernicious anemia (PA) are hypergastrinemic and total gastrin levels can be up to forty times higher than normal levels. A recent study by Varro et al, J. Clin. Invest., 95:1642-1649 (1995) has demonstrated that the hypergastrinemia associated with PA is composed of substantially elevated amidated gastrin, and moderate elevations in the precursors progastrin and glycine extended G17 (gly-G17).

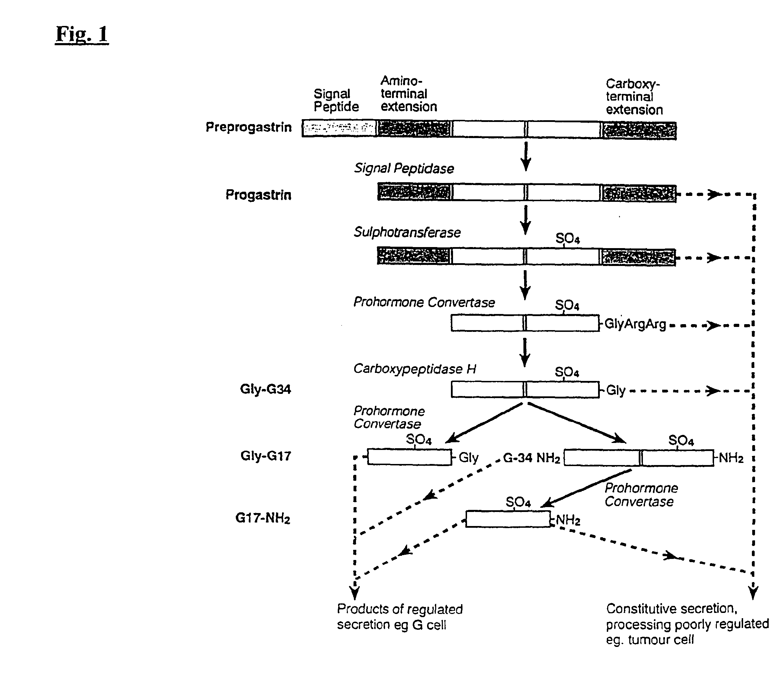

Gastrin peptides are the products of extensive post-translational processing as outlined in Fig. 1. The first translation product of a single mRNA of 0.7 kb is the 101 amino acid precursor preprogastrin. This peptide is translocated into the lumen of the rough endoplasmic reticulum where it is converted into the progastrin peptide. The progastrin moves through the secretory pathway to the golgi stack, and is sulfated at Tyr⁸⁷ prior to endoproteolytic cleavage and maturation in the secretory granules. As a consequence, progastrin is processed to give G34 from dibasic cleavage at sites Arg⁵⁷ Arg⁵⁸ and Arg⁹⁴ Arg⁹⁵ and to give G17 from dibasic cleavage at sites Lys⁷⁴ Lys⁷⁵ and Arg⁹⁴ Arg⁹⁵. While prehormone convertase 2 (PC2) producing G17 is located primarily in the gastric antrum, the prohormone convertases PC1/PC3 producing G34 are located in the duodenum. The dibasic cleavage residues are removed by carboxypeptidase H (CPH) producing Gly⁹³ extended gastrins serving as substrates for the amidation enzyme, PAM (peptidylglycine α-amidating monoxgenase).

Amidated G17 gastrin appears to be a conversion product of G34 NH₄ which is an amidation product of Gly-G34. Gly G17 has been thought as a second endpoint of progastrin processing.

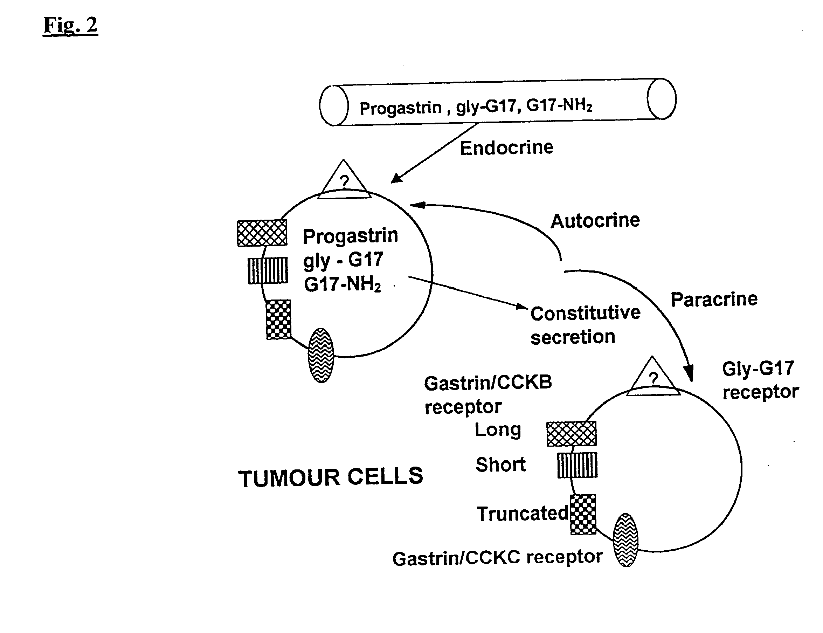

Gastrin effects on tumor cells are via endocrine, paracrine, autocrine and intracrine pathways (Fig. 2) where, however, not all receptor types have been characterized. It is known that exogenous gastrin stimulates gastric and colorectal tumor cells and tumor cell lines.

Most PA patients have endocrine hyperplasia in the gastric corpus and fundus. There is a significant positive correlation between the degree of hypergastrinemia and the number of enterochromaffin-like (ECL) cells. However, the histological type of ECL cell hyperplasia is not dependent on the degree of hypergastrinemia as there is no significant difference in the gastrin levels in patients with linear or nodular hyperplasia. Once diagnosed, despite continuing elevated gastrin levels, the ECL cell hyperplasia appears to remain stable.

The prevalence rate of gastric carcinoid in endoscopically examined PA patients' ranges from 4 to 7%. Patients with carcinoid are diagnosed as having PA 10 years earlier than the average PA patient. This precedes the diagnosis of the carcinoid tumor by a mean of 10 to 12 years. There is no predictive sign for the occurrence of gastric carcinoids in patients with PA, though mean serum gastrin levels are higher in carcinoid compared to ECL hyperplasia [Brinton et al, Brit. J. Cancer, 59:810-813 (1989)]. The stimulus to undergo malignant transformation is thought to be provided by the autoantibodies present. The tumors appear hormonally dependent. Patients who have undergone antrectomy in order to correct hypergastrinemia, have demonstrated disappearance of hyperplastic polyps, carcinoids or agyrophil micronodules diagnosed endoscopically and/or histologically. The demonstration of complete resolution of ECL-cell carcinoids after antrectomy in some patients confirms the potency of hypergastrinemia as a trophic principle for fundic ECL-cells.

In PA, evidence for an effect of the associated hypergastrinemia on other cancers in the gastrointestinal tract comes only from epidemiological studies. Several studies have looked at the incidence of colorectal cancer in PA. A slight increase in the prevalence of colorectal cancer in the first five years after diagnosis of PA has been reported [Talley et al, Annals Int. Medicine, 111:738-742 (1989)].

Studies have also demonstrated an increased prevalence (approximately 7%) of gastric adenocarcinoma in PA. Hypergastrinemia may be responsible for this observed increase. Even though a correlation to serum gastrin levels cannot be found in the majority of patients with gastric cancer, a correlation to chronic atrophic gastritis is always present.

The epidemiological studies have failed to show a consistent increase in the incidence of colorectal cancer in PA. This may be due to the deficient design of the studies. In each analysis the PA patients were compared to unscreened controls from the general population. A proportion of the controls may be expected to be hypergastrinemic due to either *Helicobacter pylori* infection, atrophic gastritis or following administration of a proton pump inhibitor. The apparent failure to show an increase in the incidence of tumors could be explained by the action of gastrin - it acts as a mitogen not a mutagen. However, as gastrin promotes the proliferation of the normal colonic mucosa, there may be an increased chance of a spontaneous mutation, which would affect tumor incidence.

A single study performed recently has looked at the proliferation rate of cells of the normal colon in patients with PA compared to normal controls [Talley et al, cited above]. The control patients were normogastrinemic and had no colonic abnormalities assessed by colonoscopy or barium enema. Using 5'-bromodeoxyuridine to provide a proliferation index, the percentage of proliferating cells in the entire crypts was similar in both groups. In the PA group there was a significantly higher labeling frequency in the upper two fifths of the glands (p<0.01). Movement of the proliferative compartment is seen in individuals at high risk of cancer.

Long-term treatment with omeprazole is known to induce ECL cell hyperplasia which is related to the serum gastrin level. Chronic hypergastrinemia-related carcinoid tumors of the stomach have been reported in certain animals test subjects, e.g. rats, although not yet confirmed in the human [Sobhani et al, Gastroenterology, 105:22-30 (1993)].

Proton pump inhibitors cause a twofold to fourfold increase in fasting and postprandial plasma gastrin concentrations. The increase in fasting hypergastrinemia occurs within a few months of starting therapy. Occasionally, markedly elevated gastrin levels (10 fold) may develop during long term treatment with omeprazole, e.g., 20-60mg/day. Gastrin levels stabilize after a few months of therapy even if the dose of omeprazole is decreased from 40mg to 20 mg daily [Sontag et al, Gastroenterology, 102:109-118(1992)].

The growth of gastric endocrine cells has been extensively monitored in patients treated with 20 to 40mg omeprazole daily for up to eight years. No significant quantitative changes of the antral G- and D- cells have been found even after years of high-dose omeprazole treatment. In comparison, the G-cell volume in rats doubled both qualitatively and quantitatively after four weeks of treatment with omeprazole [Tielemans et al, Gastroenterology, 96:723-729 (1989)]. Only patients with the highest serum gastrin levels (>240 pg/ml, four times the upper limit of normal) showed an increase in gastric ECL-cell volume density between the third and fifth year of therapy. This data supports earlier findings that an increase of the ECL-cell volume density is correlated to elevated fasting serum gastrin levels. In addition, linear and nodular hyperplasia was confined to the group of patients with the highest serum gastrin levels. Dysplasia was not been seen in any patient.

A correlation between different grades of atrophy of the oxyntic mucosa and ECL cell growth has been established. In patients receiving 40mg of omeprazole daily for eight years, it was found that the prevalence of micronodular hyperplasia in superficial corpus gastritis was low, e.g., 3.6%, increasing to 19.6% in interstitial gastritis and to 48% in atrophic gastritis. This relationship between atrophic gastritis and micronodular hyperplasia may partially be explained by condensation of the endocrine cells caused by atrophy of the gastric glands and thus may not represent true hyperplasia. Therefore, excessive long-lasting hypergastrinemia induced by omeprazole leads to only linear and simple hyperplasia. In patients with atrophic gastritis or those with a genetic predisposition, hypergastrinemia gives rise to micronodular hyperplasia under the chronic treatment.

ECL hyperplasia in animal models occurs following the administration of omeprazole. The relative growth of both exocrine and endocrine cells produced by hypergastrinemia varies between species. For example, administration of omeprazole, (400µmol/kg, 14mg/kg) to mice for 10 weeks resulted in a threefold increase in plasma gastrin during treatment. Furthermore, the stomach weight increased by 34% and the ECL density by 37% at the end of treatment. The same dose has been found in rats to increase the gastrin levels 10-fold, resulting in the same general trophic effect (increase of stomach and mucosal weight) as in mice, but the ECL cell density increases by about 300%. The significance of this imbalance in the trophic effect of gastrin on the exocrine cells and ECL cells for the development of carcinoids in rats is not known.

Several studies have investigated the effect of hypergastrinemia on normal colonic epithelial cells. The majority of these studies have induced hypergastrinemia by omeprazole administration or as a result of antral exclusion and have produced conflicting results. The effect of long-term (1-year) treatment of female rats with high dose daily omeprazole (400µmol/kg, 14mg/kg) which led to 15 fold increase in gastrin compared to controls was examined [Sundler et al, in Proc. The First Interntional Symposium on Omeprazole. K. O. Borg et al eds, AB Hässle (1986)]. The mucosal thickness of the colonic mucosa and the number of chromogranin-A-containing endocrine cells were unaffected by the omeprazole-induced hypergastrinemia. However, the same animals developed a modest and stable antral gastrin cell hyperplasia. Similarly, Oscarson demonstrated that long-term changes in endogenous gastrin concentration produced by fundectomy (resulting in a 3.5 fold gastrin elevation) did not result in colonic mucosal trophic effects.

In contrast, significantly enhanced proliferation of colonic mucosa in omeprazole treated rats compared to controls was demonstrated [Pawlikowski e tal, Hormone & Metab. Res., 21:89-91 (1989)]. Short term hypergastrinemia induced colonic mucosal proliferation as well as chronic endogenous hypergastrinemia were demonstrated in rats [McGregor et al, Annals Surg., 195:219-223 (1982)]. Chronic hypergastrinemia was achieved by antral exclusion and short term hypergastrinemia was achieved by pentagastrin administration (2mg/kg) every 12 hours for 48 hours prior to sacrifice. Tissue content and synthesis of DNA, RNA, and protein were all markedly increased by both endogenous gastrin and exogenous pentagastrin. The stimulation by gastrin was significantly stronger than that of pentagastrin. Using the metaphase-arrest technique it has also been have shown that an enhanced mitotic activity of the colonic mucosal cells in rats treated with omeprazole compared to controls [Lewinski et al].

More compelling evidence for a trophic role of gastrin has been provided by the development of gastrin deficient transgenic mice. These mice are incapable of producing gastrin mRNA and the gastrin peptide. This deficiency has allowed studies on the effect of gastrin on the growth and development of the gastrointestinal tract.

Combining histology and immunohistochemical techniques, together with bromodeoxyuridine incorporation, the effect of exogenous gastrin on colonic architecture was assessed. The gastrin deficient mice had histologically normal colons. A decreased proliferation labeling index (2.97% ± 0.52%) was noted in such mice compared with wild-type animals (4.71% ± 0.44%; P < 0.01). The conclusion from these observations is that gastrin is trophic for the normal colonic mucosa.

According to Tang et al. (1996) carcinoid tumors from the Mastomys rodent during progression lose response to exogenous hypergastrinemia but have up-regulated expression of TGFU. As TGFÜ autocrine pathway potentially acts in a co-operative way with the gastrin autocrine pathway [Howell et al, (1997)], the lack of response of the carcinoids to exogenous gastrin may reflect the increasing activity of the gastrin autocrine pathway. The gastrin gene is apparently activated to rather a lower extent in adenomas than adenocarcinomas.

The conflicting results produced by the above studies may in part be explained by Wang et al, J. Clin. Invest., 98:1918-1929 (1996), in which evidence was provided that progastrin, once thought to be an inert precursor, also has a trophic effect on colonic mucosa. The study included the use of transgenic mice containing a human gastrin (hGAS) minigene, that expresses abundant human gastrin mRNA and human progastrin in the liver. The hepatocytes are unable to process this peptide to the mature amidated form, resulting in markedly elevated serum progastrin levels and normal amidated gastrin levels. The result was a marked increase in the bromodeoxyuridine labeling index of the colon, but not the gastric mucosa, in hGAS mice compared to age-matched, wild- type control mice. This study suggests that progastrin may contribute to colonic mucosal proliferation *in vivo.* Therefore, in conditions of hypergastrinemia not only may the degree of hypergastrinemia be important but the particular gastrin peptide which is elevated may also play a significant role. Normal colonic epithelial cells do not express classical gastrin/CCKB receptors so the action of gastrin must be mediated by an uncharacterized receptor that mediates the action of gastrin precursors.

As stated above, gastrin acts as a mitogen, and thus would not be expected to cause a cell to mutate. This hypothesis which has been confirmed in transgenic hGAS mouse studies. However, if the mucosa has an enhanced proliferation rate, there may be an increased chance of sporadic mutation. The only example of malignant change in animal models occurring in the presence of hypergastrinemia is carcinoid in rats following long term omeprazole administration. Although this finding is particular to rats, and no other animal model produces spontaneous carcinoids, it was felt that omeprazole may have a direct carcinogenic effect. However, the proton pump inhibitor class of drugs that produce hypergastrinemia, ECL hyperplasia and ECL carcinoids in the rat, has tested negatively for genotoxicity. Subsequent studies have shown that it is not a specific drug that leads to carcinoid formation; carcinoids can also be produced by feeding with 2000mg/kg ranitidine, loxitidine, the hypolipidemic agent clofibrate and by 75% corpectomy, all of which produce hypergastrinemia. The mediator role of gastrin was confirmed when it was shown that antrectomy in rats prevents omeprazole induced ECL cell hyperplasia. The formation of carcinoids in rats simply in the presence of hypergastrinemia may be due to their genetic background.

There is no reported evidence of hypergastrinemia producing spontaneous tumors at other sites in the gastrointestinal tract. In humans, it is evident that an additional factor may be required for ECL cells to progress from simple hyperplasia to carcinoid. In PA, the additional factor is possibly supplied by the presence of autoantibodies.

Once the cell has been transformed, exogenous gastrin can continue to promote growth. This effect may be enhanced by gastrin/CCKB receptors which are expressed de novo on adenomas. The exact point in the adenoma-carcinoma transformation sequence at which the gastrin/CCKB receptor and autocrine gastrin are expressed is not yet known. Hypergastrinemia may increase this transforming progression through the stages of the adenoma-carcinoma sequence.

In addition, treatment with agents directed against excess production of gastric acid has been found to induce parietal cell hyperplasia and hypertrophy. Recent cases were reported to suggest a correlation between gastric acid-inhibitory treatment by either proton pump inhibitors, such as omeprazole, lansoprazole, or histamine H₂ receptor inhibiting agents, such as ranitidine or cimetidine, and the occurrence of fundic gland polyps (FGP).

A therapeutic method for selectively immunologically neutralizing the biological activity of the gastrin hormone would provide an effective means to control or prevent the physiopathological changes resulting from hypergastrinemia.

As disclosed in co-assigned U.S. Patents Nos. 5,609,870; 5,607,676; 5,622,702; 5,468,494; and 5,023,077, immunization against the G17 and G34 gastrin forms can effect neutralization of serum gastrin. The immunogenic constructs of this invention include an aminoterminal (1-9) G17 peptide or an aminoterminal (1-6) G34 peptide conjugated via a peptide spacer to an immunogenic carrier. The preferred G17 sequence is pyro-Glu-Gly-Pro-Trp-Leu-Glu-Glu-Glu-Glu [SEQ ID NO: 1] and the preferred G34 sequence is pGlu-Leu-Gly-Pro-Gln-Gly-Arg-Pro-Pro-Pro-Pro-Cys [SEQ ID NO: 2]. The preferred spacer in both constructs is a Ser-peptide (Ser-Ser-Pro-Pro-Pro-Pro-Cys [SEQ ID NO: 3]). The preferred immunogenic carrier is diphtheria toxoid, tetanus toxoid, keylimpet hemocyanin, and bovine serum albumin (BSA). The gastrin immunogen is defined as a conjugate of the pGlu- Gly-Pro-Trp-Leu-Glu-Glu-Glu-Glu [SEQ ID NO: 1] peptide sequence, with an amino acid spacer linked to an immunogenic carrier. The preferred gastrin immunogen is defined as a conjugate of the (1-9) amino terminal (pGlu-Gly-Pro-Trp-Leu-Glu-Glu-Glu-Glu [SEQ ID NO: 1]) peptide which is linked by peptide spacer to diphtheria toxoid. It is further known that the gastrin immunogen preparation is also effective for inhibiting the incompletely processed or progastrin type gastrin precursors which may be bound to the cell membrane of a gastrin producing cell.

There is a need in the art for compositions and methods to effectively treat hypergastrinemia.

### SUMMARY OF THE INVENTION

The present invention is directed to the treatment for control or prevention of gastrointestinal disorders such as hypergastrinemia by administering a gastrin immunogen preparation to an afflicted mammal or human.

A preferred embodiment of the treatment is directed to the control or prevention of hypergastrinemia due to pernicious anemia.

Another preferred embodiment of this invention is directed to the treatment for control or prevention of gastrointestinal side effects due to antiulcer agents such as proton pump inhibitors or histamine H₂ receptor blocking agents or antagonists.

It is another preferred embodiment of this invention to treat hypergastrinemia related to colorectal disorders or diseases by immunization with gastrin immunogen against gastrin peptide G17, G34, amidated gastrin and progastrin. In this context, the anti-G17 immunogen as described in U. S. Patent Nos. 5,609,870; 5,468,494; 5,785,970 and in the co-assigned patent application 08/798,423 has been found to provide an effective agent to stimulate anti-G17 antibodies which cross-react with Gly extended G17 (G17-Gly), amidated G17 (G17 NH₂) so as to be suitable for treating gastrointestinal tumors which are responsive to these gastrin peptides. The '423 application is incorporated herewith by reference in its entirety.

It is a special advantage of the present invention to provide a specific immunogen or antibody to target the specific protein which results in hypergastrinemia. For example, Gly G17 and G17 NH₂ can be neutralized with an anti-G17 immunogen composition, such as G17 (1-9) Ser DT, while G34 can be neutralized with anti-G34 (1-17) immunogens.

Moreover, G17 and G34 can be neutralized by anti-G34 (13-22) and anti-G34 (17-31) immunogens which generate antibodies able to cross-react with both gastrin epitopes. Passive immunization can be effected by the specific antibodies generated by immunogens against the various G17 and G34 epitope. These antibodies will either react specifically and separately with the G17 or G34 epitopes or react with both such gastrin epitopes together.

It is an especially preferred embodiment of this invention to treat or pre-treat with gastrin immunogen-type immunization a patient or mammal who is under chronic or long term treatment with the proton pump inhibitor, omeprazole or lansoprazole. A further embodiment provides passive immunization with anti-G17 antibodies which may be humanized to treat hypergastrinemia. A perfected combination treatment of hypergastrinemia and concomitant excess product of gastric acid involves administration of proton inhibitors or H₂ histamine receptor blockers.

### BRIEF DESCRIPTION OF THE DRAWINGS

Fig. 1 illustrates the processing of the gastrin precursor to the mature gastrin forms;
Fig. 2 illustrates the various pathways of gastrin activity;
Fig. 3 illustrates the structural aspects of a gastrin immunogen.

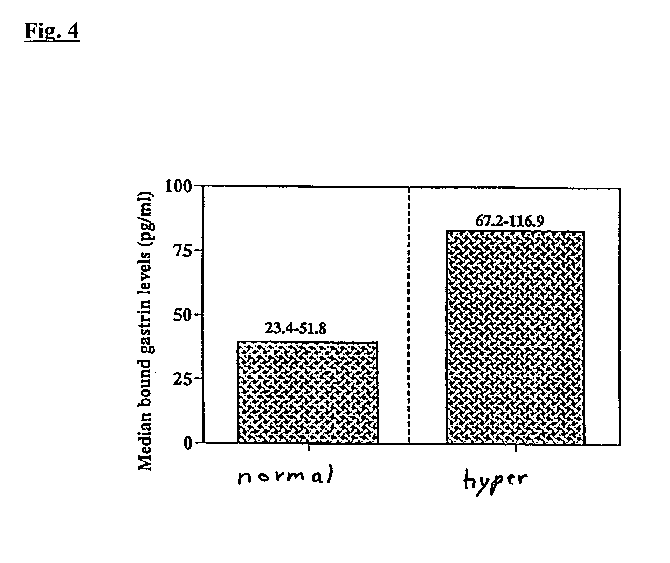

Fig. 4 illustrates bound gastrin (median) in normal and hypergastrinemic subjects (the immunogen control group did not have bound serum gastrin above the 10 pg/ml detection limit).
Fig. 5 illustrates percentage survival of experimental hypergastrinemia mice treated with gastrin immunogen compared to controls.
Fig. 6 depicts the mean proliferation index of gastrin associated tumors in Min mice under treatment with gastrin immunogen.
Fig. 7 illustrates the timed levels of antibody in Min mice immunized with G17 (1-9):DT.
Fig. 8 compares the Min mouse anti-G17 (1-9):DT antibody levels in response to hG17-DT immunogen + vehicle, hG17-DT immunogen + omeprazole, vehicle only, omeprazole only, positive and negative controls.
Fig. 9 compares Min mouse serum G17 levels when immunized with 1) hG17-DT immunogen plus vehicle (Free G17), 2) plus vehicle (Bound G17), 3) plus omeprazole (free G17) and 4) plus omeprazole (Bound G17).

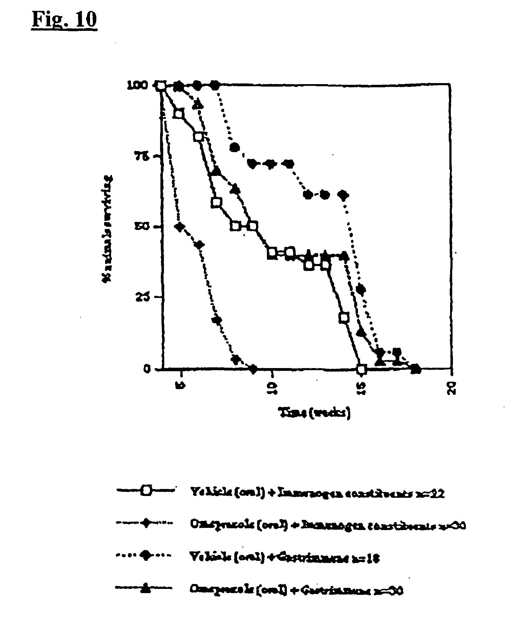

Fig. 10 illustrates the percent animals surviving after treatment with oral vehicle plus blank immunogen (n=22); omeprazole (n=18) plus blank immunogen (n=30), oral vehicle plus gastrin immunogen (n=18); and omeprazole hG17-DT immunogen (n=30).
Fig. 11 shows the displacement of labelled G17 from anti-N-terminal gastrin (from rabbit anti-human G7 antiserum) by G17, Gly-G17, and G34 as described in Example 5.
Fig. 12 shows the displacement of labelled G17 from anti-C-terminal gastrin (from rabbit anti-human G7 antiserum) by G17, Gly-G17, and G34 as described in Example 5.

### DETAILED DESCRIPTION OF THE INVENTION

As described above, immunogenic constructs useful in this invention include an aminoterminal (1-9) G17 peptide or an aminoterminal (1-6) G34 peptide conjugated via a peptide spacer to an immunogenic carrier. The preferred G17 sequence is pyro-Glu-Gly-Pro-Trp-Leu-Glu-Glu-Glu-Glu [SEQ ID NO: 1] and the preferred G34 sequence is pGlu-Leu-Gly-Pro-Gin-Gly-Arg-Pro-Pro-Pro-Pro-Cys [SEQ ID NO: 2]. The preferred spacer in both constructs is a Ser-peptide (Ser-Ser-Pro-Pro-Pro-Pro-Cys [SEQ ID NO: 3]). The preferred immunogenic carrier is diphtheria toxoid, tetanus toxoid, keylimpet hemocyanin, and bovine serum albumin (BSA). The gastrin immunogen is defined as a conjugate of the pGlu- Gly-Pro-Trp-Leu-Glu-Glu-Glu-Glu [SEQ ID NO: 1] peptide sequence, with an amino acid spacer linked to an immunogenic carrier. The preferred gastrin immunogen is defined as a conjugate of the (1-9) amino terminal (pGlu-Gly-Pro-Trp-Leu-Glu-Glu-Glu-Glu [SEQ ID NO: 1]) peptide which is linked by peptide spacer to diphtheria toxoid.

In addition to the above-named anti-gastrin immunogens, one of the generating antibodies binding to both G17 and G34 comprises a conjugate of the 7 amino acids of C-terminal G17 amino acid sequence 11-17-DT. This sequence is E-A-Y-G-W-M-D-NH₂ [SEQ ID NO: 4]. An inhibition of G34 and G17 induced gastric acid production in perfused rat stomachs was observed after an intravenous injection of 1ml of rabbit Anti-C terminal G17 (11-17)-DT antisera.

In order to explore whether gastrin may promote progression to malignancy in existing pre-malignant conditions, studies were undertaken, for example, in the multiple intestinal neoplasia or so-called *Min* mouse model of familial adenomatous polyposis (FAP). The mice have a germline mutation in their APC gene which leads to multiple intestinal neoplasia. Hypergastrinemia which was induced by use of the proton pump inhibitor, omeprazole, has been now found to increase progression to malignancy in *Min* mice, reducing their median survival from approximately 10 weeks to 6 weeks. Examination of the proliferation of tumors from *Min* mice exposed to elevated gastrin levels revealed by bromodeoxyuridine incorporation that proliferation was increased.

In addition to the proliferative effects of serum-associated gastrin, acting in an endocrine manner to increase proliferation, expression of the gastrin gene has also been shown in the colonic mucosa in pre-malignant condition. In the transgenic APC1628 mouse, the gastrin gene is activated in both the normal colonic mucosa and the malignant epithelium [Smith et al, Brit. J. Surg., 84:706 (1997)]. This has recently been confirmed by applicants by both immunocytochemistry and at the gene level in the *Min* mouse. In addition, activation of the gastrin gene has been found in human adenomas [Smith et al, (1997) cited above]. Thus, a gastrin-mediated autocrine/paracrine pathway may also be operational in the pre-malignant scenario.

Effect of gastrin neutralization on the progression of the adenoma:carcinoma sequence in the *Min* mouse model of familial adenomatous polyposis has been observed. Specifically, both serum-associated gastrin and gastrin present with the colonic epithelium may contribute towards the progression cascade in the *Min* mouse model of FAP. Accordingly, the effect of gastrin neutralization on *Min* mouse survival was determined.

The *Min* mice used in the study were bred within the Academic Unit of Cancer Studies at Nottingham University (U.K.) on a C57/BL background. As the homozygous state is lethal and female *Min* mice do not lactate, a *Min* heterozygote is bred with a female wild type and 1:4 offspring have the *Min* genotype. The *Min* positive mice were then placed into each arm of the therapy on an ongoing basis.

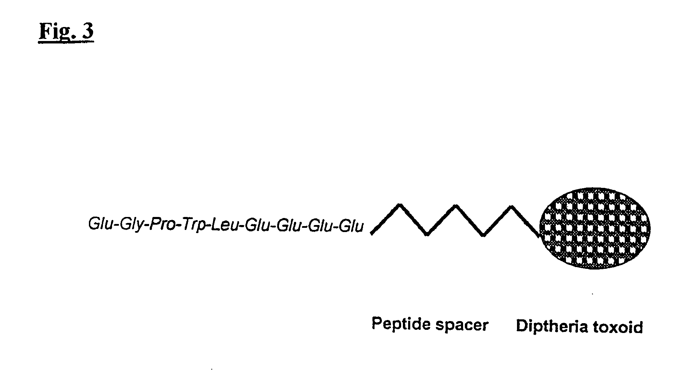

The immunization with hG17-DT immunogen (G-17 conjugate) (Fig. 3), is effective in neutralizing circulatory gastrin levels as well as tissue bound precursors or incompletely processed progastrin.

Using exogenous anti-G17 antibodies which can be in humanized form, a patient can be preimmunized against hypergastrinemia or hypergastrinemic effects caused by treatment with proton inhibitors (omeprazole, lansoprazole, or pantoprazole) or H₂ receptor blockers (ranitidine, cimetidine, formatidine or nizatidine). Humanized antibodies may be prepared by techniques known in the art.

The hG17-DT conjugated immunogen or method of preparation are disclosed in U.S. Patent No. 5,609,870, U.S. Patent No. 5,468,494 and U.S. Patent No. 5,023,077 which are incorporated by reference in this specification in their entirety. See, also, Examples 3 and 4 below.

The endpoint of the treatment, which has previously been validated by Moser et al. (1990), is at the terminal stage of the disease, when the mice have a large tumor burden, blood is lost in the stools, and the animals become anemic.

The following examples are provided for illustration only.

### Example 1

Gastrin neutralization was achieved by using an immunogen, i.e. the gastrin immunogen preparation, which is composed of the amino terminal domain of gastrin-17 linked, via an amino acid spacer, to diphtheria toxoid which acts as the immunogenic carrier. The antibodies raised by virtue of the design of the immunogen, cross-react with both amidated and glycine-extended gastrin-17, known proliferative forms of gastrin. *Min* mice were immunized s.c. with the hG17-DT immunogen (100 mg/mouse) at week 4, with subsequent injections at 3 weekly intervals. Serum antibody titres are known to rise within 2 weeks of the first immunization at levels with an antigen binding capacity of > 10 ⁻⁹ M. The hG17-DT immunogen was administered to mice at 4 weeks of age to examine its effect on mice with an established tumor burden. Control mice received immunogen constituents without the active peptide.

The presence of anti-gastrin antibodies within the serum of gastrin immunogen-immunized mice was confirmed by using an ELISA capture assay. To confirm the presence of antibody-bound gastrin, serum was taken from immunized mice, antibody:antigen complexes were purified, uncomplexed by boiling and the bound gastrin measured by RIA.

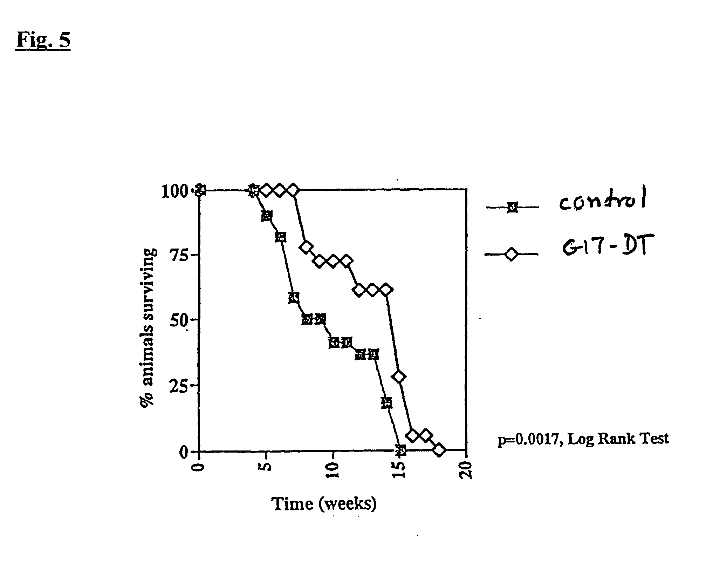

Bound gastrin levels were not measurable in animals immunized with control immunogen. The bound gastrin levels in the gastrin immunogen-immunized mice were 37pg/ml. In hypergastrinemic mice, with a 3-4 fold increase in serum gastrin levels, the bound gastrin levels was 148.3pg/ml which highlights the capacity of the antibodies raised in the serum for gastrin neutralization (Fig. 5).

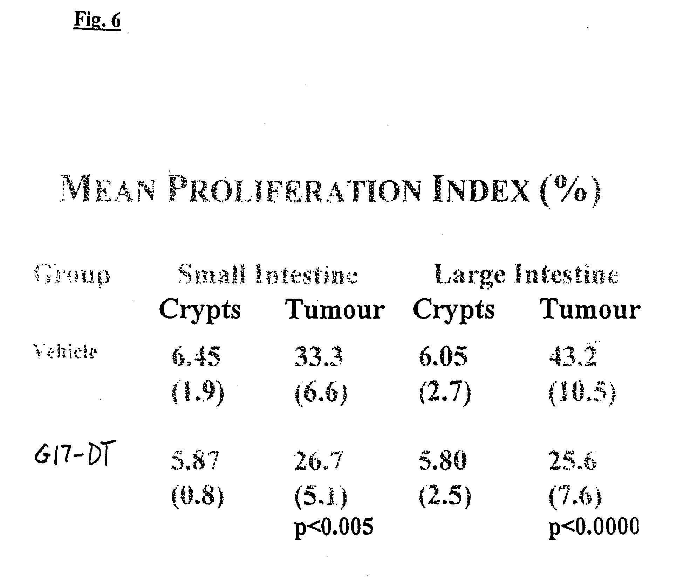

There was a significant effect on survival, with the median survival in the control immunogen treated group being 8 weeks compared to 15 for the gastrin immunogen treated mice (Fig. 6). Log rank test p=0.0017. However, there is a sharp decline in the survival of the mice after 14-15 weeks.

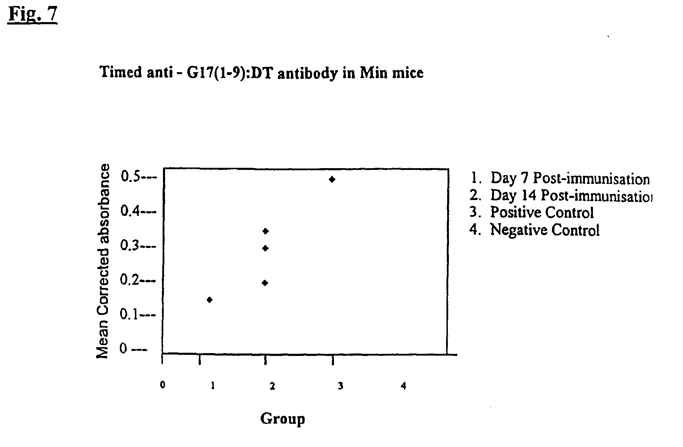

One hour prior to termination, mice were injected with DNA analogue bromodeoxyuridine in order to determine the comparative in situ proliferation activity. The samples were formalin fixed, paraffin embedded, sections were cut and stained with an anti-bromodeoxyuridine antibody. There was no significant difference in the proliferation of crypts from both the small and large intestine due to gastrin immunogen treatment (Fig. 7). The proliferation of the small intestinal tumors were significantly inhibited by 19.8%, as was the proliferation of the large intestinal tumors which was inhibited by 41%.

The normal colonic mucosa is sensitive to the proliferative effects of hypergastrinemia involving both amidated gastrin and progastrin (Wang, et al ., 1997; Renga et al., 1997). APC1638 and *Min* mice (both have mutations in their APC gene) have an activated gastrin gene in both normal and malignant colonic mucosa, unlike the corresponding wild type mice. *Min* mice have greater proliferation levels in normal mucosa when compared to the wild type C57/BL mouse.

Hypergastrinemia induced by treatment with high daily doses of omeprazole decreases the survival of *Min* mice, which is partially reversed when co-administered with gastrin immunogen. There is an initial 2 week window when hypergastrinemia is unopposed due to lack of anti-gastrin antibodies. This effect is completely reversed when omeprazole treatment is delayed for 2 weeks allowing anti-gastrin antibody titers to rise prior to onset of hypergastrinemia.

Administration of gastrin immunogen has no effect on survival of mice of increased age (6-12 weeks). However, there is a significant effect on survival of mice immunized at an earlier age (4 weeks). Therefore, the results suggest a stage through which the mice pass and after which an anti-proliferative effect is not enough to suppress malignant progression. The subject mice thus would respond to serum-associated gastrin until onset of the gastrin autocrine pathway at a later age which may be more refractory to gastrin immunogen-induced antibodies.

When gastrin immunogen was given 2 weeks prior to omeprazole treatment a complete reversal of the survival effect of omeprazole-induced hypergastrinemia on *Min* mice was observed. This was confirmed when it was shown that the omeprazole + gastrin immunogen treated group was not significantly different from the vehicle control (p=0.1103).

Anti-G17 antibodies had previously been shown to be detectable in 4 week old *Min* mice by week 2 (Fig. 6) following immunization with gastrin immunogen. Determination of the anti-G17 antibody levels at the termination of the study revealed a variation in both groups as measured by specific absorbance using an ELISA assay. Some suppression of the levels of anti- G-17 antibodies in mice co-treated with omeprazole was observed perhaps due to the neutralization of the omeprazole-induced hypergastrinemia (Fig. 7, p=0.02, Mann Whitney, comparing anti-G17 (1-9):DT antibody levels in vehicle and omeprazole-treated groups).

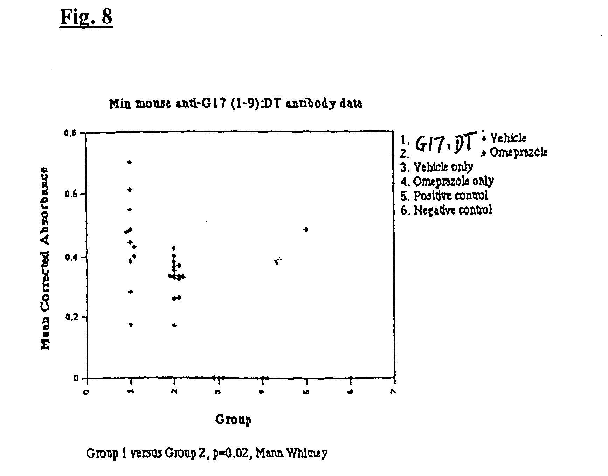

The *Min* mouse anti-G17(1-9):DT antibody data are illustrated in Fig. 5. Measurement of the free and bound serum carboxy-amidated gastrin levels in immunized animals revealed a mean free gastrin level of 28.9pg/ml and a bound level of36.7pg/ml (Fig. 8).

In the mice treated with gastrin immunogen + omeprazole, the free gastrin level was 45.6pg/ml (1.5 fold increase compared to animals immunized with gastrin immunogen alone, p=0.0135, mann Whitney). The bound gastrin level in the gastrin immunogen + omeprazole treated mice was 148.3pg/ml which was significantly increased when compared to animals treated with gastrin immunogen alone (2 animals had bound gastrin levels of greater than 200pg/ml) p=0.00001 when compared to bound gastrin levels in the gastrin immunogen treated group and p=0.00001 when compared to free gastrin levels in the gastrin immunogen + omeprazole group, [Mann Whitney]. The *Min* mouse serum G17 data are illustrated in Fig. 8. The data are shown in Table 1.

**Table 1**

| Group | Mean | SD | Statistics (Mann Whitney) |
|---|---|---|---|
| 1. | 28.9 | 12.7 | 1vs2 p=0.191 |
| 2. | 36.7 | 15.4 | 1vs3 p=0.0135 |
| 3. | 45.6 | 19.2 | 2vs4 p=0.00001 |
| 4. | 148.3 | 170.9 | 3vs4 p=0.00001 |

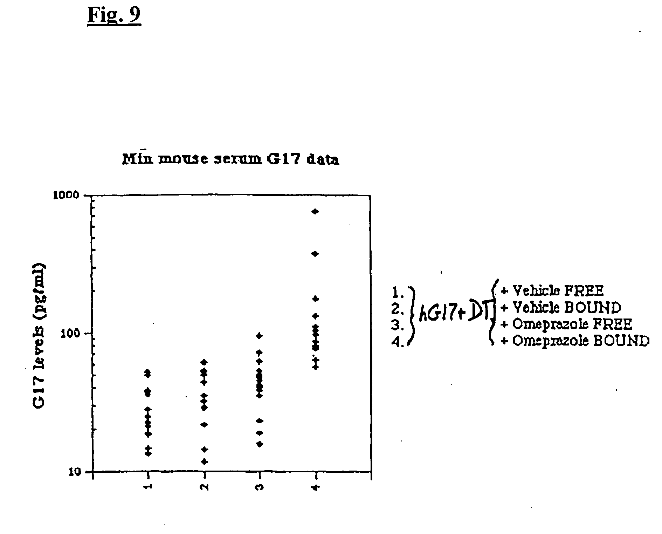

G17-DT immunogen administered alone induced a significant effect on survival (Fig. 9, p=0.0017). The effect on survival was greatest in the initial phase of the experiment with time to 50% survival being 9.5 weeks for the vehicle control and 14.5 weeks for immunogen treated. G17-DT immunogen only induced this beneficial effect on survival until week 14, following which there was an exponential drop

### Example 2

As described below, the effect of omeprazole induced hypergastrinemia on the progression of the intestinal neoplasia was further studied in the *Min* (multiple intestinal neoplasia) mouse model of polyposis coli.
Confirmed *Min* ⁺ genotype mice were randomized into 4 groups:
Group 1. OME 75mg/kg daily oral treatment
Group 2. OME + GSI 100mg oral dose/ mouse day 0 and every 3 weeks
Group 3. Oral vehicle + control immunogen
Group 4. Oral vehicle - control immunogen

Serum gastrin level was measured by RIA. Prior to end of treatment, proliferative index was determined by the bromodeoxyuridine method.
Group 1. 236pg/ml of serum gastrin
Group 4. 67pg/ml of serum gastrin

Group 1 hypergastrinemia significantly decreased survival compared to control (p=0.0001, log rank test) with mice in control group having a 50% survival of 16 weeks compared to 8 weeks in the omeprazole treated group. HG17-DT immunogen induced formation of serum antibodies with antigen binding capacity of > 20mg/ml resulting in effective neutralization of the hypergastrinemic state. Gastrin neutralization resulted in a reversal of the survival disadvantage induced by omeprazole (p=0.0017).

The hypergastrinemic mice had enhanced proliferation of normal colonic mucosa. It was found that 9.46% proliferating cells increased to 20.1%, p<0.05, Mann-Whitney and colonic neoplasia (22.3% increased 35.0%, p<0.01). Thus, the level of this experimental hypergastrinemia was in the range attained in the humans on a maintenance dose of omeprazole and resulted in enhanced progression through the adenoma:carcinoma sequence. Moreover, gastrin was confirmed as the mediator inducing a state of hyper-proliferation within both normal and neoplastic colonic epithelium. This data demonstrate the need and effectiveness for controlling hypergastrinemia on pre-malignant colon by gastrin immunogen immunization.

Polypoid carcinomas have been established *in vitro* from the large and small intestine of *Min* mice. Proliferation was assessed by a tetrazolium-based colorimetric ELISA assay. It was found that proliferation of both tumor types was not increased by amidated gastrin, but the large intestinal tumor was modestly stimulated by glycine-extended gastrin.

Gastrin immunogen immunization significantly affects the survival of *Min* mice when administered early in their life span. Moreover, the proliferation index of tumors in the large intestine was more extensively inhibited by the G17-DT immunogen than that of tumors arising in the small intestine. In this context, tumors from the large intestine of *Min* mice appear to be more sensitive to the proliferative effects of GlyG17 than tumors from the small intestine. This could be both serum-associated and tumor-associated GlyG17, the latter being due to activation of the gastrin gene in these tumors.

Immunological inhibition by G17-DT immunogen at the terminal stage of the adenoma:carcinoma sequence was not as effective. As the small intestinal tumors are the most prolific in terms of number and total tumor burden, an inhibitory effect on proliferation of less than 20% discussed above, may not be great enough to stabilize progression.

Thus, it is clear that the above-described results lead to the following conclusions:
1. The MIN mouse over-expresses the APC gene, the mutation apparently responsible for the adenoma formation a pre-cancerous stage.
2. The adenomas are sensitive to gastrin stimulation especially in early stages and/or young mice.
3. Administration of proton pump inhibitors or H₂ blockers as described, causes hypergastrinemia, hyperproliferation of adenous as and consequently shortened survival.
4. Immunization contemporaneous with omeprazole partially reverses the deleterious effect on survival. Immunization with G17-DT immunogen 2 weeks prior to the proton pump inhibitor administration resulted in complete reversal of the deleterious effects of the drug. In this regimen, the rise in antibody titers coincided with the start of the proton pump inhibitor treatment.

Treatment with the G17-DT immunogen as described is useful in reversing hyper-gastrinemic states induced by a variety of conditions, including, PA, H. pylori, atrophic gastritis, total or partial gastrectomy, treatment with proton pump inhibitors or H₂ blockers. The G17-DT immunogen is potentially effective in protecting the subject mammal, including humans, from induction of cancers responsive to gastrin (colon, stomach, pancreas, and liver).

Immunization against gastrin according to the present method of using hG17-DT induces an effective immune response in humans such that it reduces serum gastrin levels in hypergastrinemic patients to normal or lower levels.

Treatment of PA patients exhibiting hypergastrinemia with immunization (active or passive) against gastrin can be applied alone or in combination with a secondary step of anti-gastric acid administration proton pump inhibitors such as omeprazole or lansoprazole, as well as H₂ receptor blocking agents, such as rantidine cimetidine, fomatidine or nizatidine.

### Example 3

Immunogens capable of inducing specific immune responses to either G17 or to G34 were prepared by standard solid state synthesis methods. Each peptide was characterized as to amino acid content and purity.

Peptides with the following amino acid sequences were synthesized:
Peptide 1 - Human G17(1-6) ("hG17(6)"): pGlu-Gly-Pro-Trp-Leu-Glu-Arg-Pro-Pro-Pro-Pro-Cys
Peptide 2 - Human G17(1-5) ("hG17(5)"): pGlu-Gly-Pro-Trp-Leu-Arg-Pro-Pro-Pro-Pro-Cys
Peptide 3 - Human G17(1-4) ("hG17(4)"): pGlu-Gly-Pro-Trp-Arg-Pro-Pro-Pro-Pro-Cys
Peptide 4 - Rat G17(1-6) ("rG17(6)"): pGlu-Arg-Pro-Pro-Leu-Glu-Arg-Pro-Pro-Pro-Pro-Cys
Peptide 5 - Human G34(1-6) ("hG34(6)"): pGlu-Leu-Gly-Pro-Gln-Gly-Arg-Pro-Pro-Pro-Pro-Cys
Peptide 6 - Human G34(13-22) ("hG34/G17 combination"): Asp-Pro-Ser-Lys-Lys-Gln-Gly-Pro-Trp-Leu-Pro-Pro-Pro-Pro-Cys

Each of these peptides were conjugated to amino groups present on a carrier such as Diphtheria toxoid ("DT") via the terminal peptide cysteine residue utilizing heterobifunctional linking agents containing a succinimidyl ester at one end and maleimide at the other end of the linking agent.

To accomplish the linkage between any of Peptides 1-6 above and the carrier, the dry peptide was dissolved in 0.1 M Sodium Phosphate Buffer, pH 8.0, with a thirty molar excess of dithiothreitol ("DTT"). The solution was stirred under a water saturated nitrogen gas atmosphere for four hours. The peptide containing reduced cysteine was separated from the other components by chromatography over a G10 Sephadex column equilibrated with 0.2 M Acetic acid. The peptide was lyophilized and stored under vacuum until used. The carrier was activated by treatment with the heterobifunctional linking agent, e.g., Epsilon-maleimidocaproic acid N-hydroxysuccinimide ester, ("EMCS"), in proportions sufficient to achieve activation of approximately 25 free amino groups per 10⁵ molecular weight of carrier. In the specific instance of diphtheria toxoid, this amounted to the addition of 6.18 mg of EMCS (purity 75%) to each 20 mg of diphtheria toxoid.

Activation of diphtheria toxoid was accomplished by dissolving each 20 mg aliquot of diphtheria toxoid in 1 ml of 0.2 M Sodium Phosphate Buffer, pH 6.45. Aliquots of 6.18 mg EMCS were dissolved into 0.2 ml of Dimethyl Formamide ("DMF"). Under darkened conditions, the EMCS was added dropwise in 50 microliter ("ul") amounts to the DT with stirring. After 2 hours of incubation in darkness, the mixture was chromatographed on a G50 Sephadex column equilibrated with 0.1 M Sodium Citrate buffer, pH 6.0, containing 0.1 mM EDTA.

Fractions containing the EMCS activated diphtheria toxoid were concentrated over a PM 10 ultrafiltration membrane under conditions of darkness. The protein content of the concentrate was determined by either the Lowry or Bradford methods. The EMCS content of the carrier was determined by incubation of the activated carrier with cysteine-HCI followed by reaction with 10 mM of Elman's Reagent 5,5'dithio-bis (2-nitrobenzoic acid) 10 mM. The optical density difference between a blank tube containing cysteine-HCl and the sample tube containing cysteine-HCl and carrier was translated into EMCS group content by using the molar extinction coefficient of 13.6 x 10³ for 5-thio-2-nitro benzoic acid at 412 nm.

The reduced cysteine content (-SH) of the peptide was also determined utilizing Elman's Reagent. Approximately 1 mg of peptide was dissolved in 1 ml of nitrogen gas saturated water and a 0.1 ml aliquot of this solution was reacted with Elman's Reagent. Utilizing the molar extinction coefficient of 5-thio-2-nitro-benzoic acid (13.6 x 10³), the free cysteine -SH was calculated. An amount of peptide containing sufficient free -SH to react with each of the 25 EMCs activated amino groups on the carrier was dissolved in 0.1 M Sodium Citrate Buffer, pH 6.0, containing 0.1 mM EDTA, and added dropwise to the EMCS activated carrier under darkened conditions. After all the peptide solution had been added to the carrier, the mixture was incubated overnight in the dark under a water saturated nitrogen gas atmosphere.

The conjugate of the peptide linked to the carrier via EMCS is separated from other components of the mixture by chromatography over a G50 Sephadex column equilibrated with 0.2 M Ammonium Bicarbonate. The conjugate eluted in the column void volume is lyophilized and stored desiccated at 20°C until used.

The conjugate may be characterized as to peptide content by a number of methods known to those skilled in the art including weight gain, amino acid analysis, etc. Conjugates of these peptides and diphtheria toxoid produced by these methods were determined to have 20-25 moles of peptide per 10⁵ MW of carrier and all were considered suitable as immunogens for immunization of test animals.

### Example 4

Peptide hG17(1-9)-Ser9 was prepared by standard solid state synthesis methods. That peptide contains an amino terminal immunomimic of hG17 followed by a carboxy terminal spacer. This peptide comprises a 9 amino acid immunomimic of hG17 (pGlu-Gly-Pro-Trp-Leu-Glu-Glu-Glu-Glu-) followed by the "Ser" spacer (-Ser-Ser-Pro-Pro-Pro-Pro-Cys) attached to amino acid number 9 of the hG17 immunomimic.

The peptide was conjugated to amino groups present on the Diphtheria Toxoid ("DT") immunogenic carder via the terminal peptide cysteine residue utilizing heterobifunctional linking agents containing a succinimidyl ester at one end and maleimide at the other end of the linking agent essentially as described in Example 4.

The immunogenic constructs of this invention include an aminoterminal (1-9) G17 peptide or an aminoterminal (1-6) G34 peptide conjugated via a peptide spacer to an immunogenic carrier. The preferred G17 sequence is pyro-Glu-Gly-Pro-Trp-Leu-Glu-Glu-Glu-Glu and the preferred G34 sequence is pGlu-Leu-Gly-Pro-Gin-Gly-Arg-Pro-Pro-Pro-Pro-Cys. The preferred spacer in both constructs is a Ser-peptide (Ser-Ser-Pro-Pro-Pro-Pro-Cys). The preferred immunogenic carrier is diphtheria toxoid, tetanus toxoid, keylimpet hemocyanin, and bovine serum albumin (BSA). The gastrin immunogen is defined as a conjugate of the pGlu- Gly-Pro-Trp-Leu-Glu-Glu-Glu-Glu peptide sequence, with an amino acid spacer linked to an immunogenic carrier. The preferred gastrin immunogen is defined as a conjugate of the (1-9) amino terminal (pGlu-Gly-Pro-Trp-Leu-Glu-Glu-Glu-Glu) peptide which is linked by peptide spacer to diphtheria toxoid.

### Example 5

These experiments demonstrate that the immunogen induces antisera that bind to amidated G17 and glycine-extended G17, but not to G34. Specifically, this experiment demonstrates the gastrin-specificity of an antiserum raised by anti-G17 immunization of rabbits.

Antisera were absorbed onto a solid phase at a concentration of 100 µg/ml and displacement was determined in a competitive assay with a fixed concentration of radiolabelled G17 (100 pg/ml) and increasing concentrations of unlabelled ligands (1-25,000 pg/ml).

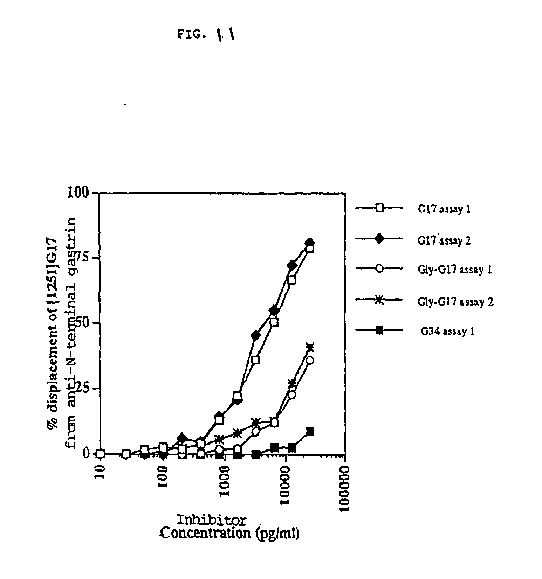

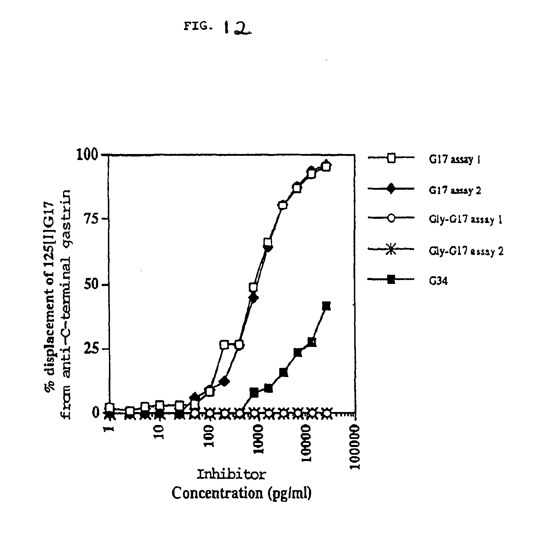

Figs. 11 and 12 show the displacement of [¹²⁵I]G17 from rabbit anti-human G17 antiserum by G17, G17-Gly and G34. The antiserum used in the test depicted in Fig. 11 was obtained form animals immunized with G17(1-9):DT and was specific for the N-terminal end of G17; the antiserum for Fig. 12 was specific for the C-terminal end of G17. G17 displaced radiolabelled G17 from both antisera preparations with a 50% inhibitory concentration (IC₅₀) of 3 500 pg/ml for the rabbit anti-human G17(1-9):DT (N-terminal) and 800 pg/ml for the rabbit anti-G17 (C-terminal). Glycine-extended G17 did not displace radiolabelled G17 from the C-terminal specific antiserum, but did from the N-terminal specific antiserum (IC₂₅ 12,000 pg/ml), demonstrating that the glycine-extended G17 binds to N-terminal specific antiserum, but not to C-terminal specific antiserum. G34 displaced radiolabelled G17 from the C-terminal (IC₂₅ 500 pg/ml), but not the N-terminal specific antiserum, demonstrating the specificity of the G17(1-9):DT antiserum for G17 and glycine-extended G17 and not to G34.

This invention and its preferred embodiments have been described in detail. One skilled in the art, upon consideration of this disclosure, may make modifications and improvements within the scope of this invention.

## Claims

1. A method for treating and/or preventing hypergastrinemia comprising administering to a patient in need thereof a therapeutically effective amount of an antigastrin immunogenic composition, comprising a G17 or G34 peptide fragment or a combination thereof linked by an amino acid spacer to an immunogenic carrier.

2. The method of claim 1, wherein the spacer is a Ser peptide spacer.

3. The method of claim 1, wherein the immunogenic carrier is selected from the group consisting of diphtheria toxoid, tetanus toxoid, and keylimpet hemocyanin.

4. The method of claim 1, wherein the hypergastrinemia is associated with pernicious anemia or administration of a substance which results in increased gastrin levels.

5. A method for treating and/or preventing hypergastrinemia comprising administering to a patient in need thereof a therapeutically effectively amount of anti-G17 antibodies.

6. The method of claim 5, wherein the hypergastrinemia is associated with pernicious anemia or administration of a substance which results in increased gastrin levels.

7. A method for treating tumors associated with hypergastrinemia comprising administering to a gastrin related tumor bearing patient an anti-gastrin immunogen or anti-gastrin antibodies.

8. Use of a therapeutically effective amount of an antigastrin immunogenic composition, comprising a G17 or G34 peptide fragment or a combination thereof linked by an amino acid spacer to an immunogenic carrier in the preparation of a medicament for treating and/or preventing hypergastrinemia in a patient.

9. Use according to claim 8, wherein the spacer is a Ser peptide spacer.

10. Use according to claim 8, wherein the immunogenic carrier is selected from the group consisting of diphtheria toxoid, tetanus toxoid, and keylimpet hemocyanin.

11. Use according to claim 8, wherein the hypergastrinemia is associated with pernicious anemia or administration of a substance which results in increased gastrin levels.

12. Use of a therapeutically effective amount of anti-G17 antibodies in the preparation of a medicament for treating and/or preventing hypergastrinemia in a patient.

13. Use according to claim 12, wherein the hypergastrinemia is associated with pernicious anemia or administration of a substance which results in increased gastrin levels.

14. Use of an anti-gastrin immunogen or anti-gastrin antibodies in the preparation of a medicament for treating tumors associated with hypergastrinemia in a gastrin related tumor bearing patient.


SEQUENCE LISTING

<110> APHTON CORPORATION

<120> Prevention and Treatment of Hypergastrinemia

<130> 1102865-0035

<140> US
<141> 1999-05-14

<150> US 60/085,714
<151> 1999-05-15

<160> 4

<170> PatentIn Ver. 2.0

<210> 1
<211> 9
<212> PRT
<213> human or synthetic

<220>
<221> MOD_RES
<222> (1)
<223> pyroglutamic acid

<400> 1
Glu Gly Pro Trp Leu Glu Glu Glu Glu
 1               5

<210> 2
<211> 12
<212> PRT
<213> human or synthetic peptide

<220>
<221> MOD_RES
<222> (1)
<223> pyroglutamic acid

<400> 2
Glu Leu Gly Pro Gln Gly Arg Pro Pro Pro Pro Cys
 1               5                   10

<210> 3
<211> 7
<212> PRT
<213> Artificial Sequence

<220>
<223> Description of Artificial Sequence:Synthetic
      peptide

<400> 3
Ser Ser Pro Pro Pro Pro Cys
  1               5


<210> 4
<211> 7
<212> PRT
<213> human or synthetic peptide

<220>
<221> MOD_RES
<222> (7)
<223> AMIDATION

<400> 4
Glu Ala Tyr Gly Trp Met Asp
  1               5